# EUROPÄISCHE PATENTSCHRIFT

(11) **EP 0 660 729 B1**
(45) Veröffentlichungstag und Bekanntmachung des Hinweises auf die Patenterteilung: **22.07.1998**
(21) Anmeldenummer: 94913091.8
(22) Anmeldetag: 30.03.1994
(51) Int. Cl.: A61M 5/168

(54) **VORRICHTUNG ZUR INFUSIONSÜBERWACHUNG**
INFUSION MONITORING DEVICE
DISPOSITIF POUR LE CONTROLE DE PERFUSION

(30) Priorität: 20.07.1993 DE 4324230; 29.10.1993 DE 4337017; 29.11.1993 DE 4340536
(43) Veröffentlichungstag der Anmeldung: 05.07.1995
(73) Patentinhaber: Lang, Volker, Prof. Dr., D-82131 Gauting (DE)
(72) Erfinder: Lang, Volker, Prof. Dr., D-82131 Gauting (DE)
(74) Vertreter: Laufhütte, Dieter, Dr.-Ing.
(86) Internationale Anmeldenummer: EP9401004
(87) Internationale Veröffentlichungsnummer: WO9503079

(56) Entgegenhaltungen:
- EP-A- 0 018 649
- WO-A-87/05225
- DE-C- 3 816 128
- US-A- 3 731 679

## Beschreibung

Die Erfindung betrifft eine Vorrichtung zur Infusionsüberwachung gemäß dem Oberbegriff des Anspruchs 1.

Bei der intravenösen Infusionstherapie werden heute vor allem mit Hilfe von Schwerkraftinfusionssystemen sowie Peristaltik-, Schlauch-, Kassetten- und Spritzen-Pumpen verschiedene Infusionslösungen dem Patienten über meist nur einen intravenösen Zugang mit Hilfe von Hohlnadeln oder Venenkathetern zugeführt. Durch Bewegungen des Patienten während der Therapie kann es sowohl zu partiellen oder vollständigen Infusionsstops kommen, die teilweise nur wenige Sekunden dauern, aber auch über längere Zeit andauern können. Diese Phänomene können durch Abknicken patientennaher Infusionsschläuche oder auch der abführenden Venen, Verschiebungen der Infusionsnadel in der Vene, Anliegen der Katheterspitze an der Venenwandung, vollständige oder partielle Verlegung durch Blutgerinnsel u.a. bedingt sein.

Partielle oder vollständige Infusionsstops müssen selbstverständlich möglichst frühzeitig behandelt werden. Zwei wichtige Beispiele in diesem Zusammenhang sind die lebensnotwendige kontinuierliche Medikamentenzuführung und die Aufrechterhaltung eines lebensnotwendigen venösen Kreislaufzugangs. Bei jedem partiellen oder vollständigen Infusionsstop in eine Vene des Patienten kommt es zumindest anfänglich, durch das Weiterfördern der Infusionsapparaturen zu einem Ansteigen des Drucks im Infusionsschlauchsystem. Dieser Druckanstieg läßt sich messen und durch festlegen bestimmter Alarmgrenzen an einem Monitor zur Infusionsüberwachung nutzen. Leider erfolgt dieser Druckanstieg aber bei den heute verwendeten Infusionssystemen durch deren verhältnismäßig große Dehnbarkeit nur sehr langsam und bei kleinsten Infusionsgeschwindigkeiten eventuell erst nach einer halben Stunde oder garnicht. Die für die Überwachung auf dem Markt bis jetzt erhältlichen Geräte sind leider noch immer technisch nur begrenzt einsetzbar.

Aus der DE 38 16 128 C1 ist beispielsweise eine derartige Druckwarnanordnung für Infusionseinrichtungen gemäß dem Oberbegriff des Anspruchs 1 bekannt. Diese Vorrichtung weist insbesondere den Nachteil auf, daß sie schon bei geringen Bewegungen des Patienten mit dem angeschlossenen Infusionssystem aufgrund eines Rückstaus und kurzzeitigen Infusionsstops zu einer Fehlalarmauslösung führt.

Aus der US 3,731,679 ist ein tragbares Infusionssystem mit einem Drucksensor bekannt. Hierbei wird eine Metallelektrode zur Feststellung eines Überdrucks in die Flüssigkeitsleitung eingebracht.

Aufgabe der Erfindung ist es, ausgehend von dem gattungsgemäßen Stand der Technik eine Vorrichtung zur Infusionsüberwachung an die Hand zu geben, damit einerseits eine zuverlässige Infusionsüberwachung auch bei kleinsten Infusionsgeschwindigkeiten zuverlässig möglich ist und andererseits eine Fehlalarmauslösung weitgehend sicher verhindert wird.

Erfindungsgemäß wird diese Aufgabe ausgehend von einer gattungsgemäßen Vorrichtung durch die kennzeichnenden Merkmale des Anspruchs 1 gelöst. Demnach ist in dem Infusionsschlauch zwischen dem Abzweigpunkt der Druckübertragungsleitung und dem patientennahen freien Ende des Infusionsschlauches ein Einwegeventil angeordnet, das eine Fließrichtung zum Patienten hin gestattet.

Durch dieses Einwegeventil wird sicher verhindert, daß der aufgrund von Bewegungen des Patienten erfolgende Rückstau im Infusionsleitungssystem zur Fehlalarmauslösung führt.

Durch das Vorsehen einer Luftkammer in der Druckübertragungsleitung ist das zur Verfügung stehende Luftvolumen gegenüber herkömmlichen Systemen vergrößert. Hierdurch kann in wirksamer Weise verhindert werden, daß Flüssigkeit in die Umgebung einer den Flüssigkeitsdruck messenden Membran gelangt.

Bevorzugte Ausgestaltungen der Erfindung ergeben sich aus den sich an den Anspruch 1 anschließenden Unteransprüchen 2 bis 7.

Weitere Einzelheiten und Vorteile der Erfindung werden anhand des in der Zeichnung dargestellten Ausführungsbeispiels näher erläutert. Es zeigt die einzige Figur:
ein Ausführungsbeispiel einer Vorrichtung zur Infusionsüberwachung gemäß der vorliegenden Erfindung.

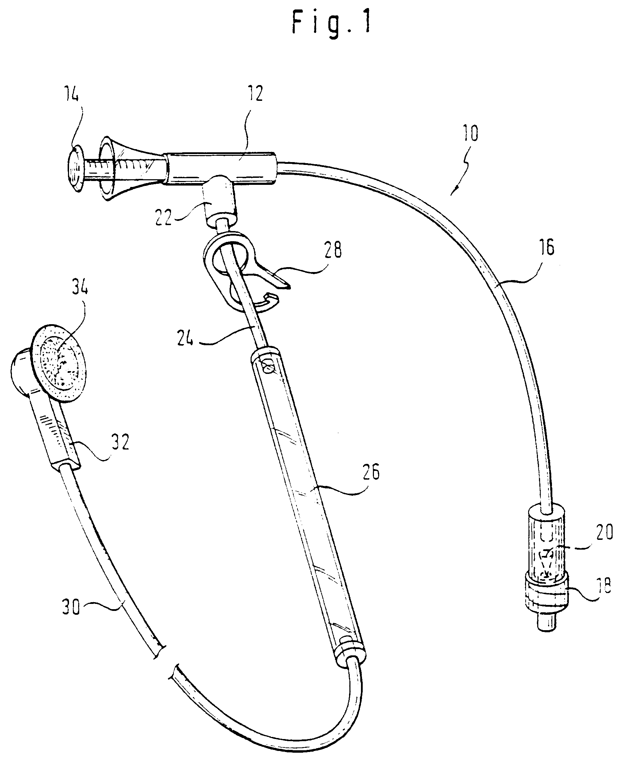

Das in Figur 1 dargestallte einfache Ausführungsbeispiel zeigt eine Vorrichtung zur Infusionsüberwachung 10 die als sterile Vorrichtung zum Einmalgebrauch für die patientennahe Infusionsduck-Überwachung benutzt werden kann. Die vorrichtung 10 besteht im wesentlichen aus einem T-Stück 12, an dessen linkem Schenkel ein weiblicher Norm-Luerkonnektor 14 zum Anschluß von Infusionsgeräten und an dessen rechtem Schenkel ein kurzer Infusionsschlauch 16 angeordnet ist, der in einem männlichen Spezial-Luerkonnektor 18 mit eingebautem Einwegeventil 20, das eine Flußrichtung zum Patienten hin zuläßt, endet. Der Luersdapter 14 dient zum Anschluß z. B. einer nicht dargestellten Hohlnadel oder eines nicht dargestellten Venenkatheters. An dem dritten T-Stück-Schenkel 22 ist ein kurzes, englumiges und dickwandiges Schlauchstück 24 angeschlossen, das vor der Mündung in eine Luftkammer 26 durch eine feststellbare Schlauchklemme 28 verschließbar ist. Die Luftkammer 26 wird beispielsweise durch ein kurzes durchsichtiges und dickwandiges, sowie weitlumiges Schlauchstück realisiert. Vom freien Ende der Luftkammer 26 führt ein durchsichtiger. dickwandiger und englumiger. ca. 50 bis 150 cm langer Druckübertragungsschlauch 30 zu einer Druckübertragungskapsel 32, die eine aufgeschweißte dünne Membran 34 enthält. Von dieser Membran kann der zu messende Infusionsdruck in bekannter Art und Weise auf einen druckempfindlichen Sensor (elektronische Druckmeßdose, federbelasteter Mikroschalter u. ä.) übertragen werden und nach elektronischer Signalverarbeitung zur optischen und akustischen Alarmsignalisierung benutzt werden.

Die Vorrichtung 10 gemäß Figur 1 kann wie folgt verwendet werden. Die Vorrichtung 10 wird nach vorherigem Abklemmen des Schlauchstücks 24 mittels der Klemme 28 über den Luerkonnektor 14 mit der Infusionsleitung verbunden und einschließlich des Spezial-Luerkonnektor 18 mit eingebautem Einwegventil 20 blasenfrei mit Infusionslösung gefüllt. Anschließend erfolgt der Anschluß an den venösen Patientenzugang (Venenkatheter, Hohlnadel). Erst nachdem die Druckübertragungskapsel 32 in die elektronische Drucküberwachungsvorrichtung eingelegt ist, was aus dem Stand der Technik bereits bekannt ist, wird die Schlauchklemme 28 geöffnet. Dadurch dringt Infusionsflüssigkeit durch das englumige, kurze Schlauckstück 24 in Abhängigkeit vom anstehenden Infusionsdruck in die weitlumige Luftkammer 26 mehr oder weniger weit ein und verdrangt dabei Luft, die über den Druckübertragungsschlauch 30 zu einer entsprechenden Vorwölbung der Druckübertragungsmembran 34 der Druckkapsel 32 zum elektronischen Drucksensor (nicht dargestellt) führt. Die Luftkammer 26 wird vorteilhaft dabei so dimensioniert, daß es bei den routinemäßigen Infusions-Druck-Messungen nicht zu einem Eintreten von Infusionsflüssigkeit in den angeschlossenen englumigen Druckübertragungsschlauch 30 kommen kann. Das Eindringen von Flüssigkeit, z. B. bei hohem Infusionsdruck, in diesen Schlauch würde bei dessen engem Lumen den Rückfluß bei nachfolgender Infusionsdruck-Normalisierung aufgrund der wirkenden Kapillarkräfte verhindern und damit zu einer unerwünschen Dauer-Fehlalarmauslösung führen.

Da jedoch bei einem alleinigen Infusions-Druck Monitoring Fehlalarme schon ausgelöst werden können bei geringen Bewegungen des zur Infusion benutzten Armes (durch kurzzeitig gestörten oder unterbrochenen venösen Abfluß der Infusionslösung oder die Entstehung hoher Druckwellen schon durch kurzes, plötzliches Abknicken der Infusionsleitung oder des intravenösen Katheters beim Patienten) werden alle diese von der Patientenseite aus über Rückflüsse entstehende Drucksteigerungen durch das Einfügen des Einwegeventils 20 eliminiert. Durch diese Maßnahme und eine in der elektronischen Druck-Alarm-Vorrichtung zusätzlich eingebaute fixe oder einstellbare Alarmverzögerung von 10 bis 60 Sekunden läßt sich eine sehr hohe Fehlalarmsicherheit erreichen, die zur Entlastung des Pflegepersonals führt, ohne dafür eine klinisch relevante Alarmverzögerung und damit eine Gefährdung des Patienten in Kauf nehmen zu müssen.

## Patentansprüche

1. Vorrichtung zur Infusionsüberwachung mit einer von dem Infusionsschlauch (16) abzweigenden Druckübertragungsleitung (30) und einer mit dieser in Verbindung stehenden Membran (34), die mit einem die Wölbungsänderung der Membran registrierenden Druckaufnehmer verbindbar ist, wobei die Druckübertragungsleitung aus englumigen Druckübertragungsschläuchen (24, 30) besteht und an das freie Ende des Druckübertragungsschlauches (30) eine Druckübertragungskapsel mit der Membran (34) angeschlossen ist,
**dadurch gekennzeichnet**,
daß in dem Infusionsschlauch (16) zwischen dem Abzweigpunkt der Druckübertragungsleitung (30) und dem patientennahen freien Ende des Infusionsschlauches (16) ein Einwegeventil (20) angeordnet ist, das eine Fließrichtung zum Patienten hin gestattet
daß die Druckübertragungsleitung ferner eine zwischengeschaltete Luftkammer (26) aufweist, und
daß die Luftkammer (26) aus einem dickwandigen, verglichen mit der Druckübertragungsleitung (24, 30) weitlumigen und vorzugsweise durchsichtigen Schlauchstück besteht.

2. Vorrichtung nach Anspruch 1, dadurch gekennzeichnet, daß sie ein über Schlauchkonnektoren in das Infusionsschlauchsystem einfügbares T-Stück (12) aufweist, über das die Druckübertragungsleitung (24) angeschlossen ist.

3. Vorrichtung nach einem der Ansprüche 1 oder 2, dadurch gekennzeichnet, daß das Einwegeventil (20) druckbelastet ist.

4. Vorrichtung nach einem der Ansprüche 1 bis 3, dadurch gekennzeichnet, daß das Einwegeventil (20) in dem patientenseitigen Schlauchkonnektor (18) integriert ist.

5. Vorrichtung nach Anspruch 2, dadurch gekennzeichnet, daß die vom T-Stück (12) abgehende Druckübertragungsleitung (24) aus einem dickwandigen, englumigen Schlauch besteht, der eine Länge zwischen 1 und 10 cm bis zur Einmündung in die Luftkammer (26) aufweist und mit einer unverlierbar angeordneten Schlauchklemme (28) versehen ist.

6. Vorrichtung nach einem der vorangehenden Ansprüche, dadurch gekennzeichnet, daß die Luftkammer (26) so ausgelegt ist, daß bei den routinemäßig gemessenen Infusionsdrucken noch keine Infusionsflüssigkeit in die abführende Druckübertragungsleitung (30) gelangt.

7. Vorrichtung nach einem der vorangehenden Ansprüche, dadurch gekennzeichnet, daß die von der Luftkammer abführende Druckübertragungsleitung (30) eine Länge von 50 bis 150 cm aufweist.

## Claims

1. Infusion monitoring system comprising a pressure transmission line (30) which branches off the infusion hose (16), and a diaphragm (34) communicating with the said line and suitable to be connected to a pressure sensor which records the curvature changes of the diaphragm, the pressure transmission line consisting of pressure transmission hoses (24, 30) with a narrow lumen and a pressure transmission capsule including the diaphragm (34) being connected to the free end of the pressure transmission hose (30),characterised in that
a single-way valve (20) allowing the infusion fluid to flow towards the patient is fitted into the infusion hose (16) between the branch point of the pressure transmission line (30) and the patient-side free end of the infusion hose (16),
the pressure transmission line further includes an intermediate air chamber (26), and
the air chamber (26) consists of a thick-walled and preferably transparent hose piece with a wide lumen compared to that of the pressure transmission line (24, 30).

2. Device according to claim 1, characterised in that it includes a T-union (12) which can be inserted into the infusion hose system using hose couplings, the T-union being used to connect the pressure transmission line (24).

3. Device according to claim 1 or 2, characterised in that the single-way valve (20) is exposed to a pressure load.

4. Device according to one of the claims 1 to 3, characterised in that the single-way valve is integrated into the patient-side hose coupling (18).

5. Device according to claim 2 characterised in that the pressure transmission line (24) leading away from the T-union (12) consists of a thick-walled hose with a narrow lumen having a length between 1 and 10 cm before reaching the air chamber (26) and including a captivated hose clip (28).

6. Device according to one of the preceding claims, characterised in that the air chamber (26) is designed in such a way that no infusion liquid can penetrate into the outgoing pressure transmission line during the routine infusion pressure measuring operations.

7. Device according to one of the preceding claims, characterised in that the pressure transmission line (30) leading away from the air chamber has a length of 50 to 150 cm.

## Revendications

1. Dispositif pour le contrôle de perfusion comprenant une conduite de transmission de pression (30) s'étendant à partir du tuyau flexible de perfusion (16), et une membrane (34) communiquant avec ladite conduite et pouvant être reliée à un capteur de pression qui répond à la variation de courbure de la membrane, la conduite de transmission de pression consistant en des tuyaux flexibles de transmission de pression (24, 30), et une capsule de transmission de pression comprenant la membrane (34) étant reliée à l'extrémité libre du tuyau flexible de transmission de pression (30),
caractérisé en ce qu' une soupape unidirectionnelle (20) permettant un flux vers le patient est disposée dans le tuyau flexible de perfusion (16) entre le point de branchement de la conduite de transmission de pression (30) et l'extrémité libre du tuyau flexible de perfusion (16), côté patient,
en ce que la conduite de transmission de pression présente de plus une chambre d'air (26) interposée et
en ce que la chambre d'air (26) consiste en une pièce de tuyau flexible avantageusement transparente, présentant une paroi épaisse et, en comparaison avec la conduite de transmission de pression, un lumen large.

2. Dispositif selon la revendication 1, caractérisé en ce qu il présente une pièce en T (12) raccordant la conduite de transmission de pression (24) et susceptible d'être insérée dans le système de tuyaux flexibles de perfusion par l'intermédiaire de raccords pour tuyaux.

3. Dispositif selon l'une quelconque des revendications 1 et 2, caractérisé en ce que la soupape unidirectionnelle (20) est pressurisée.

4. Dispositif selon l'une quelconque des revendications 1 à 3, caractérisé en ce que la soupape unidirectionnelle (20) est intégrée dans le raccord pour tuyaux (18), côté patient.

5. Dispositif selon la revendication 2, caractérisé en ce que la conduite de transmission de pression (24) s'étendant à partir de la pièce en T (12) consiste en un tuyau flexible présentant une paroi épaisse, un lumen étroit et une longueur entre 1 et 10 cm jusqu'à l'entrée de la chambre d'air (26) et en ce qu'elle est équipée d'une pince à tuyau (28) fixée de manière imperdable.

6. Dispositif selon l'une quelconque des revendications précédentes, caractérisé en ce que la chambre d'air (26) est agencée de manière que lors du mesurage de la pression de perfusion effectué par routine, aucun liquide de perfusion ne pénètre dans la conduite de transmission de pression (30) située en aval de la chambre d'air.

7. Dispositif selon l'une quelconque des revendications précédentes, caractérisé en ce que la conduite de transmission de pression (30) située en aval de la chambre d'air présente une longueur de 50 à 150 cm.
